# EUROPÄISCHE PATENTSCHRIFT

(11) **EP 1 477 159 B1**
(45) Veröffentlichungstag und Bekanntmachung des Hinweises auf die Patenterteilung: **23.07.2008**
(21) Anmeldenummer: 04008693.6
(22) Anmeldetag: 10.04.2004
(51) Int. Cl.: A61K 8/06, A61K 8/26, A61K 8/73, A61Q 17/04

(54) **Stabilisierung von Selbstbräunungsprodukten durch Schichtsilikate**
Stabilisation of self-tanning products by layered silicates
Stabilisation des produits auto-bronzants par des silicates en couches

(30) Priorität: 12.05.2003 DE 10321147
(43) Veröffentlichungstag der Anmeldung: 17.11.2004
(73) Patentinhaber: Beiersdorf AG, 20253 Hamburg (DE)
(72) Erfinder: Müller, Anja, 23843 Rümpel (DE); Eitrich, Anja, 22587 Hamburg (DE)

(56) Entgegenhaltungen:
- EP-A- 1 380 288
- WO-A-02/094205
- US-A1- 2002 031 536

## Beschreibung

Die vorliegende Erfindung betrifft selbstbräunerhaltige O/W-Emulsionen, mit einem Gehalt an Schichtsilikaten oder eine Kombination an Schichtsilikaten und Xanthan Gum..

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Die natürliche Bräunung der Haut durch Sonnenstrahlung wird verursacht durch die Bildung von braunen Farbpigmenten in der Haut. Die Epidermis enthält in ihrer untersten Schicht, der Basalschicht, neben den Basalzellen einzelne pigmentbildende Zellen, die Melanocyten. Durch UV-Licht wird in diesen Zellen die Produktion von Melanin angeregt, das in die Kerantionocycten (Hornzellen) transportiert und dort als braune Hautfarbe sichtbar wird. Diese von der Aminosäure Tyrosin ausgehende Pigmentbildung wird überwiegend durch UVB-Strahlung initiiert und als "indirekte Pigmentierung" bezeichnet. Ihre Entwicklung läuft über mehrere Tage; die so erhaltene Sonnenbräune besteht über einige Wochen.

Bei der "Direkt-Pigmentierung", die mit der Sonnenbestrahlung einsetzt, werden vorwiegend farblose Melanin-Vorstufen durch UVA-Strahlung zu dunkel gefärbten Melanin oxidiert. Da diese Oxidierung reversibel ist, führt sie zu einer nur kurz anhaltenden Hautbräunung.
Die menschliche Haut lässt sich jedoch auch künstlich bräunen. Die künstliche Bräunung der Haut lässt sich beispielsweise äußerlich mit Hilfe von Schminke und oral durch Einnahme von Carotinoiden erzeugen.

Weitaus beliebter jedoch ist die künstliche Bräunung der Haut, welche sich durch Auftragen von sogenannten Selbstbräunern erzielen lässt. Diese Verbindungen weisen als chemisches Strukturmerkmal Keto- bzw. Aldehydgruppen in Nachbarschaft zu Alkoholfunktionen auf. Diese Ketole bzw. Aldole gehören überwiegend zur Substanzklasse der Zucker. Der am häufigsten verwendete Selbstbräuner ist das 1,3-Dihydroxyaceton.

Die Verbindungen können mit den Proteinen und Aminosäuren der Hornschicht der Haut im Sinne einer Maillard-Reaktion umgesetzt werden, wobei über einen noch nicht vollständig aufgeklärten Reaktionsweg Polymerisate entstehen, die der Haut einen bräunlichen Farbton verleihen. Diese Reaktion ist nach etwa 4 bis 6 Stunden abgeschlossen; die so erzielte Bräune ist nicht abwaschbar und wird erst mit der normalen Hautabschuppung entfernt.

Handelsübliche Selbstbräunungsprodukte stellen im allgemeinen O/W-Emulsionen dar. In diesen ist die Wasserphase durch in der Kosmetik gebräuchliche Emulgatoren stabilisiert. Nachteilig ist die erforderliche zusätzliche Stabilisierung durch Carbomere. Deren Einsatz in Verbindung mit Selbstbräunern, insbesondere mit DHA, führt durch chemische Reaktion zu einer gelblichen Verfärbung der Zubereitung und zu geruchlichen Beeinträchtigungen.

Eine Alternative zum Einsatz von Carbomeren stellt der Einsatz von Xanthan Gum dar. Hierbei erhält man zwar auch stabile Produkte, man muß jedoch häufig ein unangenehms klebriges Hautgefühl in Kauf nehmen.

Die internationale Veröffentlichung WO02/094205 befasst sich mit einer Methode und einem Apparat für schnell auftragbare Make-up-Zubereitungen. Die Emulsionen sind physikalisch stabilisiert durch die Kombination aus dem Schichtsilikat Quaternium-18-hektorit mit dem Aktivator Propylencarbonat, die auf dem Träger Cyclomethicone aufgetragen sind.

Die europäische Patentschrift EP1291010 befasst sich mit einer neuen Lichtschutzfiltersubstanz, die in geringen Dosen noch hohe LSF-Werte erreicht. Es wird die physikalische Disperierung mittels Schichtsilikaten erwähnt, die organophilisiert wurden.

Diesem Nachteil des Standes der Technik abzuhelfen, war Aufgabe der vorliegenden Erfindung.

Überraschend gelöst wird die Aufgabe durch den Einsatz von Schichtsilikaten oder einer Kombination von Schichtsilikaten und Xanthan Gum, zur Stabilisierung von selbstbräunerhaltigen O/W-Emulsionen, die ein oder mehrere selbstbräunende Substanzen und gegebenenfalls weitere kosmetischen und/oder dermatologischen Wirk-, Hilfs- und Zusatzstoffe enthalten.

Durch den Einsatz von Schichtsilikaten bzw. einer Kombination von Schichtsilikaten und Xanthan Gum tritt weder eine Verfärbung noch Geruchsbeeinträchtigung der Zubereitungen auf. Auch das Hautgefühl nach der Anwendung wird im Vergleich zu Selbstbräunungszubereitungen, die dem derzeitigen Stand der Technik entsprechen, äußerst positiv bewertet.

Die erfindungsgemäßen Zubereitungen können in verschiedenen Formen vorliegen und eingesetzt werden. So können sie z. B. eine Emulsion vom Typ Öl-in-Wasser (O/W) oder eine multiple Emulsion, beispielsweise vom Typ Wasser-in-Öl-in-Wasser (W/O/W). Auch emulgatorfreie Formulierungen wie Hydrodipsersionen, Hydrogele oder eine Pickering-Emulsion sind vorteilhafte Ausführungsformen.

Vorteilhaft sind solche kosmetische und dermatologische Zubereitungen, die in der Form eines Sonnenschutzmittels vorliegen und frei von Emulgatoren und Carbomeren sind.

Die Konsistenz der Formulierungen kann von pastösen Formulierungen über fließfähige Formulierungen bis hin zu dünnflüssigen, sprühbaren Produkten reichen. Dementsprechend können Cremes, Lotionen oder Sprays formuliert werden.

Zur Anwendung werden die erfindungsgemäßen kosmetischen Zubereitungen in der für Kosmetika und Dermatika üblichen Weise auf die Haut in ausreichender Menge aufgebracht.
Durch die Anwendung lässt sich nicht nur eine gleichmäßige Hautfärbung erreichen, es lassen sich auch von Natur aus oder durch krankhafte Veränderung unterschiedlich gefärbte Hautbereiche gleichmäßig einfärben.

Als Selbstbräuner werden erfindungsgemäß vorteilhaft unter anderem eingesetzt:

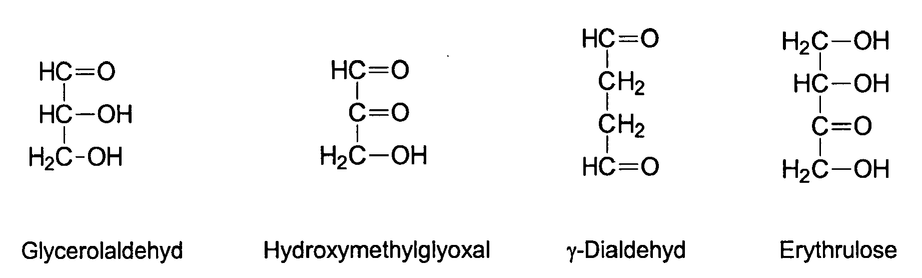

Ferner ist das 5-Hydroxy-1,4-naphtochinon (Juglon) zu nennen, das aus den Schalen frischer Walnüsse extrahiert wird 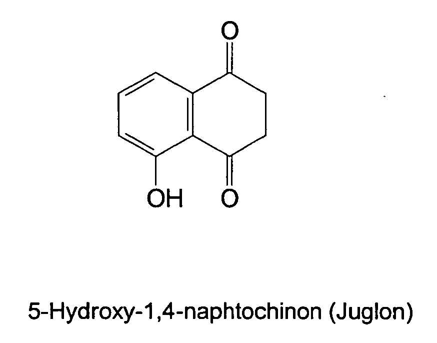 sowie das in den Henna-Blättern vorkommende 2-Hydroxy-1,4-naphtochinon (Lawson).

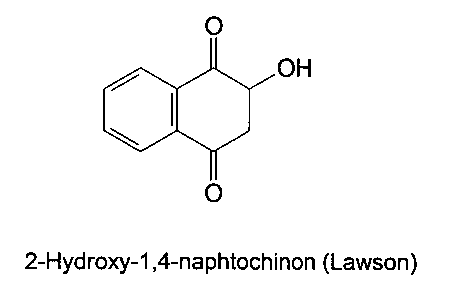

Ganz besonders bevorzugt im Sinne der Erfindung ist das 1,3-Dihydroxyaceton (DHA), ein im menschlichen Körper vorkommender dreiwertiger Zucker.

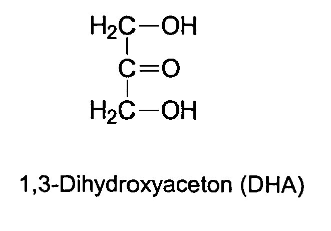

Auch 6-Aldo-D-Fructose und Ninhydrin können als erfindungsgemäße Selbstbräuner eingesetzt werden.

Als Selbstbräuner im Sinne der Erfindung sind auch Substanzen zu verstehen, die eine von Braunton abweichende Hautfärbung hervorrufen.

Es ist erfindungsgemäß von Vorteil, wenn die erfindungsgemäßen hautbräunenden kosmetischen Zubereitungen eine oder mehrere selbstbräunende Substanzen in einer Konzentration von 0,1 bis 10 Gewichts-% und besonders bevorzugt von 0,5 bis 6 Gewichts-% bezogen auf das Gesamtgewicht der Zubereitung enthalten.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose.

Silikate sind Salze und Ester (Kieselsäureester) der Orthokieselsäure [Si(OH)₄] und deren Kondensationsprodukte. Die Silikate sind nicht nur die artenreichste Klasse der Mineralien, sondern auch geologisch und technisch außerordentlich wichtig. Über 80 % der Erdkruste bestehen aus Silikaten. Schichtsilikate (Phyllosilikate, Blattsilikate) sind (idealerweise) Silikat-Strukturen mit zweidimensional unendlichen Schichten aus [SiO₄]⁴⁻-Tetraedern, wobei jedes Tetraeder über 3 Brücken-Sauerstoffe mit Nachbar-Tetraedern verbunden ist.
Chemische Formeln lassen sich für Schichtsilikate nur angenähert aufstellen, da sie ein großes lonenaustausch-Vermögen besitzen und Silizium gegen Aluminium und dieses wiederum gegen Magnesium, Fe²⁺, Fe³⁺, Zn und dergleichen ausgetauscht werden kann. Die daraus möglicherweise resultierende negative Ladung der Schichten wird in der Regel durch Kationen, insbesondere durch Na⁺ und Ca²⁺ in Zwischenschicht-Positionen ausgeglichen.
Schichtsilikate können durch reversible Einlagerung von Wasser (in der 2- bis 7-fachen Menge) und anderen Substanzen wie z. B. Alkoholen, Glykolen und dergleichen mehr aufquellen. Ihre Verwendung als Verdickungsmittel in kosmetischen Mitteln ist dementsprechend an sich bekannt. Allerdings konnte der Stand der Technik nicht den Weg zur vorliegenden Erfindung weisen.

Vorteilhafte Schichtsilikate sind beispielsweise solche, deren größte Ausdehnungsrichtung im unmodifizierten und ungequollenen Zustand im Mittel eine Länge von weniger als 10 µm hat. Beispielsweise können die mittleren Ausdehnungen der verwendeten modifizierten Schichtsilikatpartikel bei 1000 nm x 100 nm x 1 nm und darunter liegen. Die effektive Größe der modifizierten Schichtsilikatpartikel in einer kosmetischen oder dermatologischen Formulierung hängt selbstverständlich von der Menge an eingelagerten Substanzen ab.
Vorteilhafte modifizierte Schichtsilikate im Sinne der vorliegenden Erfindung sind beispielsweise modifizierte Smektite (Smectite).
Smektite sind stets sehr feinkörnige (meist < 2 mm), überwiegend als lamellenförmige, moosartige oder kugelförmige Aggregate vorkommende Dreischicht-Tonminerale (2:1-Schichtsilikate), in denen eine zentrale Schicht aus oktaedrisch koordinierten Kationen sandwichartig von 2 Schichten aus [(Si,Al)O₄]-Tetraedern umgeben ist. Smektite werden idealisiert durch die folgende Strukturformel beschrieben, worin weiß ausgefüllte Kreise Silizium- und/oder Aluminiumatome, hellgrau ausgefüllte Kreise Sauerstoffatome, dunkelgrau ausgefüllte Kreise Wasserstoffatome und schwarz ausgefüllte Kreise Aluminium-, Magnesium-, Eisenatome und/oder weitere Austauschkationen darstellen:

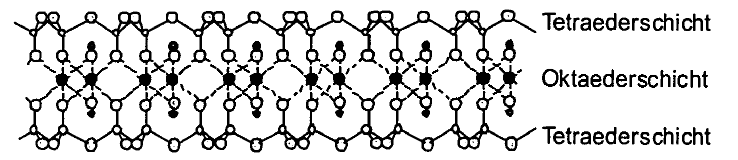

Vorteilhafte modifizierte Smektite sind z. B. modifizierte Montmorillonite. Montmorillonite werden durch die angenäherte chemische Formel Al₂[(OH)₂/Si₄O₁₀] · n H₂O bzw. Al₂O₃ · 4 SiO₂ · H₂O · n H₂O beschrieben und stellen zu den dioktaedrischen Smektiten gehörende Tonmineralien dar.
Besonders vorteilhaft im Sinne der vorliegenden Erfindung sind ferner beispielsweise modifizierte Hektorite. Hektorite gehören zu den Smektiten und haben die angenäherte chemische Formel M⁺_{0,3}(Mg_{2,7}Li_{0,3})[Si₄O₁₀(OH)₂], worin M⁺ meist Na⁺ darstellt.
Vorteilhaft im Sinne der vorliegenden Erfindung sind ferner modifizierte Bentonite. Bentonite sind Tone und Gesteine, die Smektite, vor allem Montmorillonit, als Hauptminerale enthalten. Die "Roh"-Bentonite sind entweder Calcium-Bentonite (in Großbritannien als Fuller-Erden bezeichnet) oder Natrium-Bentonite (auch: Wyoming-Bentonite).
Modifizierte Schichtsilikate im Sinne der vorliegenden Erfindung sind Schichtsilikate, insbesondere die bereits genannten Schichtsilikattypen, deren Organophilie (auch: Lipophilie) - beispielsweise durch Umsetzung mit quarternären Ammonium-Verbindungen - erhöht wurde. Solche Schichtsilikate werden auch als organophile Schichtsilikate bezeichnet.
Besonders vorteilhaft im Sinne der vorliegenden Erfindung sind sogenannte Bentone, d. h. organische Derivate von Montmorilloniten (bzw. Bentoniten) und/oder Hektoriten, die durch lonenaustausch-Reaktionen mit Alkylammonium-Basen hergestellt werden.

Vorteilhafte modifizierte Schichtsilikate im Sinne der vorliegenden Erfindung sind beispielsweise durch Umsetzung von Schichtsilikaten mit Quaternium-18 erhältlich. Quaternium-18 ist eine Mischung von quaternären Ammoniumchloridsalzen, welche durch die folgende Strukturformel beschrieben werden: 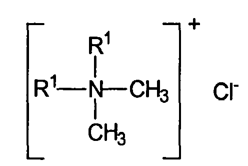 worin die Reste R¹ unabhängig voneinander gewählt werden aus hydrierten Talgresten mit einer Kettenlänge von 12 bis 20 Kohlenstoffatomen.
Erfindungsgemäß besonders bevorzugt sind Stearalkoniumhektorit, ein Reaktionsprodukt aus Hektorit und Stearalkoniumchlorid (Benzyldimethylstearylammoniumchlorid), und Quaternium-18 Hektorit, ein Reaktionsprodukt aus Hektorit und Quaternium-18, welche z. B. unter den Handelsbezeichnungen Bentone 27 und Bentone 38 bei Nordmann & Rassmann erhältlich sind.
Ganz besonders bevorzugt ist erfindungsgemäß Quaternium-90 Bentonite, ein Reaktionsprodukt aus Bentonite und Quaternium-90, das von der Firma Süd-Chemie unter dem Handelsnamen Tixogel VP-V zu beziehen ist. Die Bezeichnung deutet daraufhin, daß die Alkylreste R¹ bei diesem Produkt pflanzlichen Ursprungs sind, wodurch sich besonders vorteilhafte Eigenschaften im Sinne der vorliegenden Erfindung bzgl. Verdickung der Matrixphase und Wiederaufschüttelbarkeit des suspendierten Antitranspirant-Wirkstoffes ergeben.
So ist die Kettenlängenverteilung der Alkylreste R¹ typisch für Rohstoffquellen wie hydriertes Palmöl. Der Anteil der Alkylreste R¹ ist wie folgt, wobei sich rohstoffbedingte Schwankungen aufgrund der natürlichen Herkunft ergeben:
C₁₈H₃₇ ca. 55-65 Gew.-%, C₁₆H₃₃ ca. 30-40 Gew.-%, C₁₄H₂₉ < 5 Gew.-% Schichtsilikate und Tonmineralien wirken auf der Haut auch als Geruchsabsorber.

Die Gesamtmenge an einem oder mehreren modifizierten Schichtsilikaten und/oder Tonmineralien in der fertigen Zubereitung wird vorteilhaft aus dem Bereich von 0,05 bis 10,0 Gew.-%, bevorzugt von 0,1 bis 5 Gew.-%, besonders bevorzugt von 1,0 bis 3,0 Gew.-% gewählt, bezogen auf das Gesamtgewicht der Zubereitungen.

Xanthan Gummi (CAS-Nr. 11138-66-2), auch Xanthan genannt, stellt ein anionisches Heteropolysaccharid dar, das in der Regel durch Fermentation aus Maiszucker gebildet und als Kaliumsalz isoliert wird. Es wird von Xanthomonas campestris und einigen anderen Spezies unter aeroben Bedingungen mit einem Molekulargewicht von 2×10⁶ bis 24×10⁶ produziert. Xanthan Gummi wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan Gummi ist die Bezeichnung für das erste mikrobielle anionische Heteropolysaccharid. Es wird von Xanthomonas campestris und einigen anderen Spezies unter aeroben Bedingungen mit einem Molekulargewicht von 2-15 10⁶ produziert. Xanthan Gummi wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthan Gummis. Xanthan Gummi wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan Gummi wird anschließend getrocknet und gemahlen.

Darüber hinaus können die erfindungsgemäßen Zubereitungen vorteilhaft weitere kosmetische und/oder dermatologische Wirk-, Hilfs- und Zusatzstoffe enthalten.

Pigment. Es sind dabei alle durch die Positivliste der Kosmetikverordnung der Bundesrepublik Deutschland bzw. entsprechenden Listen der Europäischen Union zugelassenen UV-Lichtschutzfilter erfindungsgemäß vorteilhaft einsetzbar.

Vorteilhaft beträgt die Gesamtmenge der UV-Filtersubstanzen 0,1 Gew.-% bis 30 Gew.-%, vorzugsweise 0,5 bis 20 Gew.-%, insbesondere 1,0 bis 16,0 Gew.-%, bezogen auf das Gesamtgewicht der Zubereitungen, um kosmetische Zubereitungen zur Verfügung zu stellen, welche die Haut vor dem gesamten Bereich der ultravioletten Strahlung schützen. Sie können auch als Sonnenschutzmittel für die Haut dienen.

Es ist auch möglich und gegebenenfalls vorteilhaft, die erfindungsgemäßen Zubereitungen mit anderen Wirkstoffen zu kombinieren.

Die erfindungsgemäßen kosmetischen Zubereitungen können kosmetische Hilfsstoffe enthalten, wie sie üblicherweise in solchen Zubereitungen verwendet werden, z. B. Antioxidantien, Konservierungsmittel, Parfüme, antimikrobielle Wirkstoffe, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Die erfindungsgemäßen Zubereitungen enthalten vorteilhafter Weise Öle und Wachse. Vorteilhafte Öle sind hier Caprylic/Capric Triglycerid, Octyldodecanol, Butylen Glycol Dicaprylat/Dicaprat, Bis-Diglyceryl Polyacyladipat-2, Pentaerythrityl Tetraisostearat, Ricinusöl, Lanolinöl, hydriertes Polydecen, Isopropylpalmitat, Cetylpalmitat, Myristyllactat, Avocadoöl, Isostearyl Isostearat, Triisostearin, Oleyl Erucat, Jojobaöl, Dicaprylylcarbonat, Squalan, Diisostearyl Malat, Cyclomethicon, Polyisobuten, Propylencarbonat

Vorteilhafte Wachse sind Cetearylalkohol, Cetylalkohol, Oleylalkohol, Myristyl Myristat, Stearyl Beeswax + Behenyl Beeswax, Bienenwachs, Candelilla Cera, Cera Carnauba, Cera Alba, Cera Microcristallina, C16-40 Alkyl Stearat, C16-36 Alkyl Stearat, C18-38 Alkyl Hydroxystearoyl Stearat, C20-40 Alkyl Stearat, C24-40 Alkyl Stearat, Glycerylbehenat

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z. B. Hydroxyfettsäuren, Palmitinsäure, Phytinsäure, Lactoferrin), Hydroxysäuren (z. B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. Linolensäure, Linolsäure, Ölsäure), Folsäure und deren Derivate, Ubichinon und Ubichinol und deren Derivate, Vitamin C und Derivate (z. B. Ascorbylpalmitat, Mg - Ascorbylphosphat, Ascorbylacetat), Tocopherole und Derivate (z. B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,5 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 5 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 5 Gew.-%, bezogen auf das Gesamtgewicht der Formulierung, zu wählen.

Es ist insbesondere vorteilhaft, wenn die kosmetischen und/oder dermatologischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder ß-Alanin.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Auch können die erfindungsgemäßen Zubereitungen erfindungsgemäß vorteilhaft mit die Haut beruhigenden und pflegenden Substanzen versetzt sein. Hierzu zählen beispielsweise Panthenol, Allantoin, Tannin, Antihistaminika, Antiphlogistika, Glucocorticoide (z.B. Hydrocortison) sowie Pflanzenwirkstoffe wie Azulen und Bisabolol, Glycyrrhizin, Hamamelin und Pflanzenextrakte wie Kamille, aloe vera, Hamazelis, Süßholzwurzel.

Die erfindungsgemäßen Zubereitungen enthalten vorteilhafter Weise Substanzen zur Hautbefeuchtung (engl. Moisturizer). Zu den erfindungsgemäß vorteilhaften Hautbefeuchtungsmitteln zählen unter anderem Polyole wie Glycerin und Sorbit. Auch andere Hautbefeuchtungsmittel wie ethoxylierte Polyole und hydrolysierte Proteine, Komponenten des natürlichen Feuchthaltefaktors der Haut (engl. Natural Moisturizing Factor, NMF) z.B. Harnstoff, Natriumlactat und bestimmte Aminosäuren werden erfindungsgemäß vorteilhaft eingesetzt.

Erfindungsgemäß vorteilhaft beträgt die Konzentration an Hautbefeuchtungsmitteln 1 bis 20 und bevorzugt 3 bis 12 Gew.-% und ganz besonders bevorzugt 5 bis 10 Gew..-%, bezogen auf das Gesamtgewicht der Zubereitung.

Zur Anwendung werden die erfindungsgemäßen kosmetischen Zubereitungen in der für Kosmetika und Dermatika üblichen Weise auf die Haut in ausreichender Menge aufgebracht.

Nicht zuletzt ist die Verwendung von kosmetischen Zubereitungen als Selbstbräuner, Sonnenschutzmittel und/oder dekoratives Kosmetikum Teil der vorliegenden Erfindung.

Vorteilhaft sind solche kosmetische und dermatologische Zubereitungen, die in der Form eines Sonnenschutzmittels vorliegen. Vorteilhaft enthalten diese mindestens einen UVA-Filter und/oder mindestens einen UVB-Filter und/oder mindestens einen UV-Breitbandfilter und/oder mindestens ein anorganisches Pigment. Es sind dabei alle durch die Positivliste der Kosmetikverordnung der Bundesrepublik Deutschland bzw. entsprechenden Listen der Europäischen Union zugelassenen UV-Lichtschutzfilter erfindungsgemäß vorteilhaft einsetzbar.

Vorteilhaft beträgt die Gesamtmenge der UV-Filtersubstanzen 0,1 Gew.-% bis 30 Gew.-%, vorzugsweise 0,5 bis 20 Gew.-%, insbesondere 1,0 bis 16,0 Gew.-%, bezogen auf das Gesamtgewicht der Zubereitungen, um kosmetische Zubereitungen zur Verfügung zu stellen, welche die Haut vor dem gesamten Bereich der ultravioletten Strahlung schützen. Sie können auch als Sonnenschutzmittel für die Haut dienen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Make-up-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente als UV-Filter und/oder zur Stabilisierung der erfindungsgemäßen Zubereitung sind Metalloxide und/oder andere in Wasser schwerlösliche oder unlösliche Metallverbindungen, insbesondere Oxide des Titans (TiO₂), Zinks (z. B. ZnO), Eisens (z. B. Fe₂O₃), Zirkoniums (z. B. ZrO₂), Siliciums (z. B. SiO₂) bzw. Silikate (z.B. Talkum), Mangans (z. B. MnO), Aluminiums (z. B. Al₂O₃), Cers (z. B. Ce₂O₃), Mischoxide der entsprechenden Metalle sowie Abmischungen aus solchen Oxiden, sowie das Sulfat des Bariums (BaSO₄).

Im wesentlichen unerheblich für die vorliegende Erfindung ist es, in welcher der gegebenenfalls natürlich vorkommenden Modifikationen die verwendeten Metalloxide vorliegen.

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Ein solches Verfahren, das im folgenden am Beispiel von Titandioxid beschrieben wird, besteht beispielsweise darin, daß die hydrophobe Oberflächenschicht nach einer Reaktion gemäß

n TiO₂ + m (RO)₃Si-R' → n TiO₂ (oberfl.)

erzeugt wird. n und m sind dabei nach Belieben einzusetzende stöchiometrische Parameter, R und R' die gewünschten organischen Reste.

Eine weitere vorteilhafte Beschichtung der anorganische Pigmente besteht aus Dimethylpolysiloxan (auch: Dimethicone), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Besonders vorteilhaft sind TiO₂-Pigmente, beispielsweise die mit Aluminiumstearat beschichteten, unter der Handelsbezeichnung MT 100 T bei der Firma TAYCA erhältlichen.

Beschriebene beschichtete und unbeschichtete Titandioxide können im Sinne vorliegender Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.
Die erfindungsgemäßen Titandioxide zeichnen sich durch eine Primärpartikelgröße zwischen 10 nm bis 200 nm aus.

| **Handelsname** | **Coating** | **zusätzliche Bestandteile der Vordispersion** | **Hersteller** |
|---|---|---|---|
| MT-100TV | Aluminiumhydroxid Stearinsäure | - | Tayca Corporation |
| MT 100 T | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KGaA |
| Eosolex TS | Alumina, Stearinsäure | - | Merck KGaA |
| Titandioxid P25 | None | - | Degussa |
| Titandioxid T805 (Uvinul TiO₂) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |

Im Sinne der vorliegenden Erfindung sind besonders bevorzugte Titandioxide das MT-100 Z und MT-100 TV von Tayca Corporation, Eusolex T-2000 und Eusolex TS von Merck und das Titandioxid T 805 von Degussa.

Zinkoxide können im Sinne der vorliegenden Erfindung auch in Form kommerziell erhältlicher öliger oder wässriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| NanoX 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Vorteilhaft im Sinne der vorliegenden Erfindung ist ferner die Verwendung einer Mischung verschiedener Pigmenttypen sowohl innerhalb eines Kristalls, beispielsweise als Eisenmischoxid oder Talkum (Magnesiumsilicat), als auch durch Kombination mehrerer Metalloxidtypen innerhalb einer Zubereitung. Besonders vorteilhaft sind Magnesiumsilicate, beispielsweise die unter der Handelsbezeichnung Talkum Micron bei der Firma Grolmann erhältlichen.

Die Gesamtmenge an einem oder mehreren anorganischen Pigmenten in der fertigen kosmetischen Zubereitung wird vorteilhaft aus dem Bereich 0,1 Gew.-% bis 25 Gew.-% gewählt, vorzugsweise 0,5 Gew.-% bis 18 Gew.-%.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel 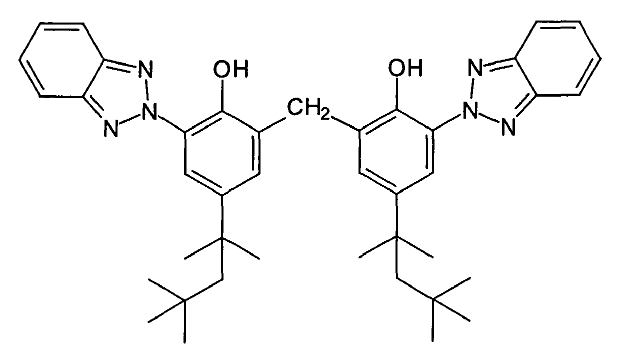 gekennzeichnet ist und unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure 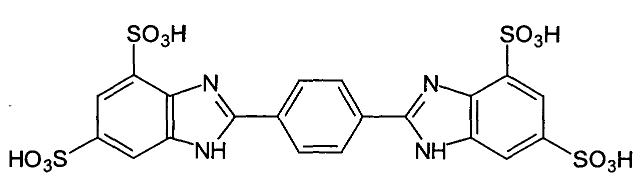 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz 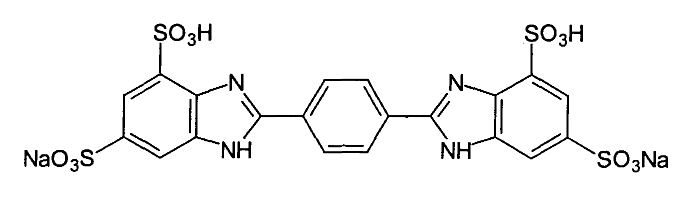 mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

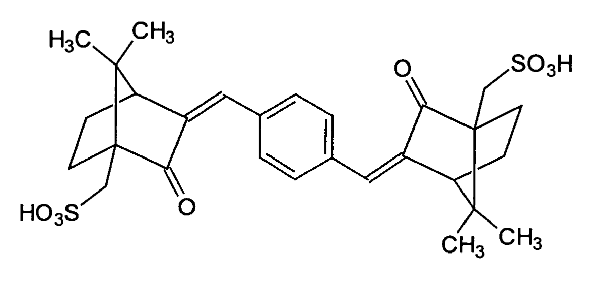

Weitere vorteilhafte UV-A-Filtersubstanzen sind Hydroxybenzophenone, die sich durch die folgende Strukturformel auszeichnen: 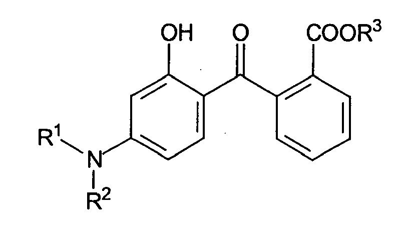 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet: 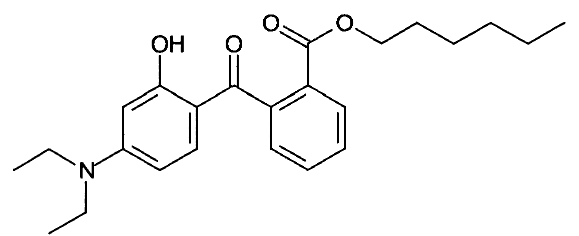 und unter dem Handelsnamen Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 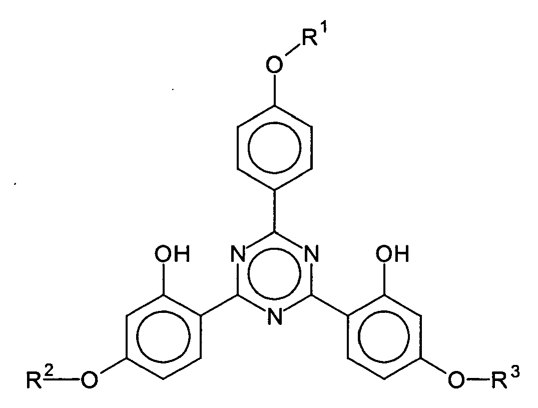 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethylhexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv 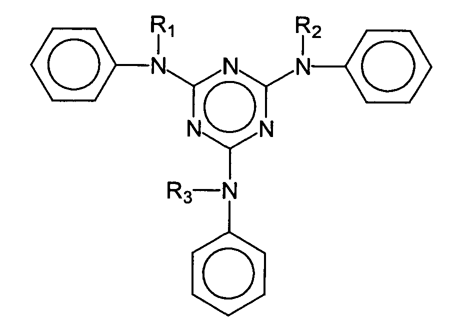 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 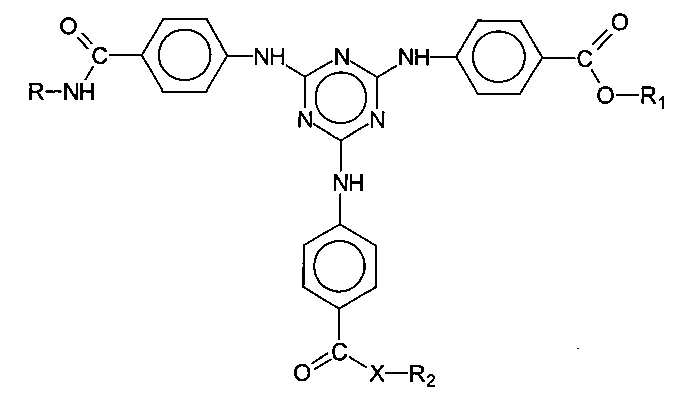 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 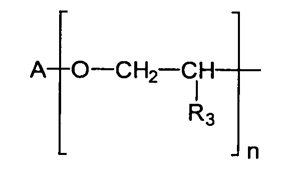 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 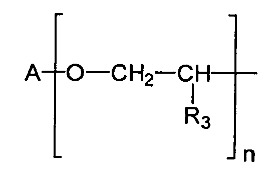 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Forme 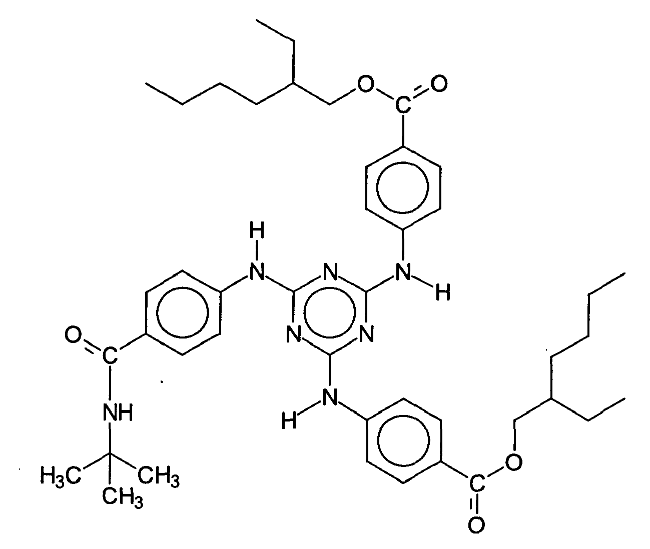 wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 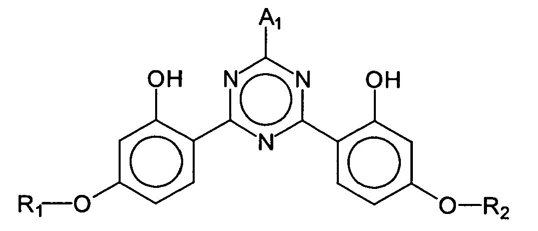 wiedergegeben wird, wobei R₁ , R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxypropyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methylpropyloxy)-2-hydroxy]-phenyl}-6-(4-methoxyphenyl)-1,3,5-triazin.
Ein vorteilhafter Breitbandfilter im Sinne der vorliegenden Erfindung ist das 2,2'-Methylenbis-(6-(2H-benzotriazol-2-yl)-4-(1,1,3,3-tetramethylbutyl)-phenol), welches durch die chemische Strukturformel 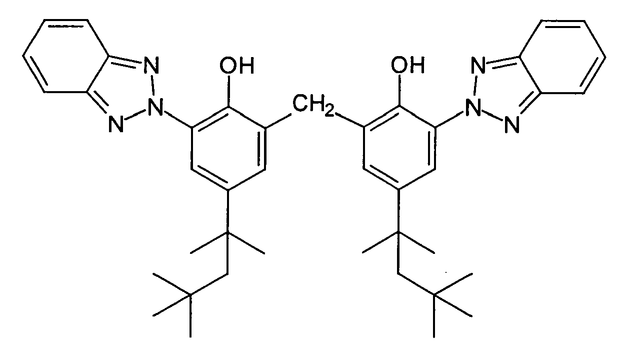 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2Hbenzotriazol-2-yl)-4-methyl-6-[2-methyl-3-[1,3,3, 3-tetramethyl-1-[(tri methylsi lyl )oxy]d i-siloxanyl]propyl]-phenol (CAS-Nr.: 155633-54-8) mit der INCI-Bezeichnung Drometrizole Trisiloxane, welches durch die chemische Strukturformel 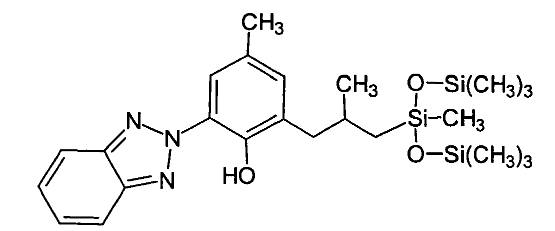 gekennzeichnet ist.

Die UV-B- und/oder Breitband-Filter können öllöslich oder wasserlöslich sein. Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-hydroxybenzoat (2-Ethylhexylsalicylat, Octylsalicylat, INCI: Octyl Salicylate), 4-Isopropylbenzylsalicylat und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/ Dimethylsiloxan - Copolymer (INCI: Dimethicodiethylbenzalmalonat) welches beispielsweise unter der Handelsbezeichnung Parsol® SLX bei Hoffmann La Roche erhältlich ist.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

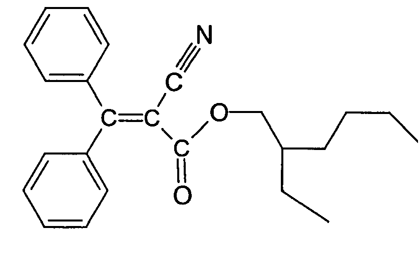

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die erfindungsgemäßen Zubereitungen können Abmischungen aus Ölen bzw. öllöslichen Substanzen und Wasser bzw. wasserlöslichen Komponenten darstellen, die durch den Zusatz von herkömmlichen Emulgatoren und/oder mikronisierten Feststoffpartikeln stabilisiert werden. Hierbei stellen die erfindungsgemäßen Zubereitungen Öl in Wasser Emulsionen dar. Besonders vorteilhaft zur Stabilisierung dieser Formulierungen sind O/W-Emulgatoren insbesondere

Erfindungsgemäß können die Zubereitungen anorganische Pigmente mit amphiphilem Charakter, die sowohl in Wasser als auch in Öl dispergierbar sind, enthalten.

Die erfindungsgemäßen Zubereitungen stellen Abmischungen aus Ölen bzw. öllöslichen Substanzen und Wasser bzw. wasserlöslichen Komponenten dar, die durch den Zusatz von mikronisierten Feststoffpartikeln stabilisiert werden und keinen Emulgator im herkömmlichen Sinn enthalten müssen. Eine Erklärungsmöglichkeit für die Stabilität dieser Zubereitungen ist, daß sich die Pigmentpartikel an die Tröpfchen der dispersen Phase anlagern und sozusagen eine mechanische Barriere bilden, die das Koaleszieren, d. h. das Zusammenfließen der Tröpfchen, verhindert.

Die erfindungsgemäßen Zubereitungen sind in jeglicher Hinsicht überaus befriedigende Präparate, die erstaunlicherweise höchst variabel in ihrem Wasser-Fettphasen-Verhältnis sind. Es war insbesondere überraschend, daß die erfindungsgemäßen Zubereitungen sich durch exzellente kosmetische Eigenschaften auszeichnen und sich daher für den Einsatz in vielen Bereichen der pflegenden und dekorativen Kosmetik anbieten. Für viele Anwendungen ist es insbesondere vorteilhaft, daß die erfindungsgemäßen Zubereitungen frei von Emulgatoren im herkömmlichen Sinne sein können. Die erfindungsgemäßen Zubereitungen sind ferner hervorragende Vehikel für verschiedenste Wirkstoffe.

Vorteilhaft ist es, den mittleren Partikeldurchmesser der verwendeten Pigmente zwischen 1 nm und 200 nm, besonders vorteilhaft zwischen 5 nm und 100 nm zu wählen.

Es ist vorteilhaft im Sinne der vorliegenden Erfindung die Metalloxide aus der Gruppe Titandioxid, Zinkoxid, Eisenoxide bzw. Eisenmischoxide, Siliciumdioxid bzw. Silicate (z. B. Talkum) zu wählen, wobei die Metalloxide sowohl einzeln als auch im Gemisch vorliegen können.

Vorteilhaft im Sinne der vorliegenden Erfindung ist es, zur Stabilisierung der Pickering-Emulsionen unbehandelte, nahezu reine Pigmentpartikel zu verwenden, insbesondere solche, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Pickering-Emulsionen im Sinne der vorliegenden Erfindung werden vorteilhaft durch anorganische Pigmente stabilisiert, die oberflächlich wasserabweisend behandelt ("gecoatet") sind, wobei gleichzeitig der amphiphile Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Es ist ferner vorteilhaft, wenngleich nicht zwingend, die erfindungsgemäßen amphiphilen anorganischen Mikropigmente mit weiteren Pigmenten zu kombinieren, beispielsweise mit Titandioxidpigmenten, die mit Octylsilanol beschichtet sind, und/oder mit Siliciumdioxidpartikeln, die oberflächlich wasserabweisend behandelt sind. Zur Kombination geeignete Siliciumdioxidpartikel sind beispielsweise sphärische Polyalkylsilsesquioxan-Partikel wie sie in der Europäischen Offenlegungsschrift 0 686 391 erwähnt werden. Solche Polyalkylsilsesquioxan-Partikel sind beispielsweise unter den Handelsbezeichnungen Aerosil R972 und Aerosil 200V bei der Firma Degussa erhältlich. Geeignete Titandioxidpartikel sind unter der Handelsbezeichnung T805 ebenfalls bei der Firma Degussa erhältlich.

Als weitere vorteilhafte Pigmente können Bornitride eingesetzt werden, beispielsweise durch die im folgenden aufgelisteten Bornitride:

| **Handelsname** | **erhältlich bei** |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN^{®} | Carborundum |
| Wacker-Bornitrid BNP | Wacker-Chemie |

Vorteilhaft ist es, den mittleren Partikeldurchmesser der verwendeten Bornitridpartikel kleiner als 20 µm, besonders vorteilhaft kleiner als 15 µm zu wählen.

Pickering-Emulsionen im Sinne der vorliegenden Erfindung werden ebenfalls vorteilhaft durch Bornitridpartikel stabilisiert, die oberflächlich wasserabweisend behandelt ("gecoatet") sind, wobei gleichzeitig der amphiphile Charakter gebildet werden bzw. erhalten bleiben soll.

Eine vorteilhafte Beschichtung der Bornitridpartikel besteht aus Dimethylpolysiloxan (Dimethicon). Vorteilhaft sind beispielsweise die bei der Firma Carborundum unter der Handelsbezeichnung Très BN^{®} UHP 1106 erhältlichen, mit Dimethicon behandelten Bornitridpartikel.

Vorteilhaft ist ferner eine Beschichtung der Bornitridpartikel mit Polymethylhydrogensiloxan, einem linearen Polysiloxan, welches auch als Methicone bezeichnet wird. Vorteilhafte, mit Methicone behandelte Bornitridpartikel sind beispielsweise die bei der Firma Carborundum unter der Handelsbezeichnung Très BN^{®} UHP 1107 erhältlichen.

Es ist ferner vorteilhaft, die erfindungsgemäßen Pickering-Emulsionen durch mikrofeine Polymerpartikel zu stabilisieren.

Vorteilhafte mikrofeine Polymerpartikel sind im Sinne der vorliegenden Erfindung beispielsweise Polycarbonate, Polyether, Polyethylen, Polypropylen, Polyvinylchlorid, Polystyrol, Polyamide, Polyacrylate und dergleichen mehr.

Erfindungsgemäß vorteilhaft sind beispielsweise mikrofeine Polyamid-Partikel, welche unter der Handelsbezeichnung SP-500 bei der Firma TORAY erhältlich sind. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε:-laurinlactam) aus ε-Laurinlactam. Vorteilhaft im Sinne der vorliegenden Erfindung sind beispielsweise Orgasol^{®} 1002 (Polyamid 6) und Orgasol^{®} 2002 (Polyamid 12) von der Firma ELF ATO-CHEM.

Weitere vorteilhafte Polymerpartikel sind mikrofeine Polymethacrylate. Solche Partikel sind beispielsweise unter der Handelsbezeichnung POLYTRAP® bei der Firma DOW CHEMICAL erhältlich.

Es ist insbesondere vorteilhaft, wenngleich nicht zwingend, wenn die verwendeten mikrofeinen Polymerpartikel oberflächlich beschichtet sind. Diese Oberflächenbehandlung kann darin bestehen, daß die Pigmente nach an sich bekannten Verfahren mit einer dünnen hydrophilen Schicht versehen werden. Vorteilhafte Beschichtungen bestehen beispielsweise aus TiO₂, ZrO₂ oder auch weiteren Polymeren, wie beispielsweise Polymethylmethacrylat.

Besonders vorteilhafte mikrofeine Polymerpartikel im Sinne der vorliegenden Erfindung sind ferner nach dem in der US-Patentschrift 4,898,913 beschriebenen Verfahren zur hydrophilen Beschichtung hydrophober Polymerpartikel erhältlich.

Vorteilhaft in ist es, den mittleren Partikeldurchmesser der verwendeten mikrofeinen Polymerpartikel kleiner als 100 µm, besonders vorteilhaft kleiner als 50 µm zu wählen. Dabei ist es im wesentlichen unerheblich, in welcher Form (Plättchen, Stäbchen, Kügelchen etc.) die verwendeten Polymerpartikel vorliegen.

Desweiteren ist es vorteilhaft, die erfindungsgemäßen Pickering-Emulsionen durch modifizierte Polysaccharide zu stabilisieren.

Modifizierte Polysaccharide im Sinne der vorliegenden Erfindung sind beispielsweise durch Umsetzung von Stärke mit mono-, bi- oder polyfunktionellen Reagenzien bzw. Oxidations-Mitteln in weitgehend polymeranalog verlaufenden Reaktionen erhältlich.

Solche Reaktionen basieren im wesentlichen auf Umwandlungen der Hydroxy-Gruppen der Polyglucane durch Veretherung, Veresterung oder selektive Oxidation. Dabei entstehen z. B. sogenannte Stärkeether und Stärkeester der allgemeinen Strukturformel 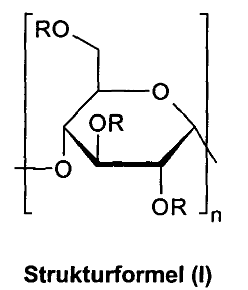 worin R beispielsweise ein Wasserstoff und/oder einen Alkyl- und/oder Aralkylrest (im Fall der Stärkeether) oder ein Wasserstoff und/oder einen organischen und/oder anorganischen Säure-Rest (im Fall der Stärkeester) darstellen kann. Stärkeether und Stärkeester sind vorteilhafte modifizierte Polysaccharide im Sinne der vorliegenden Erfindung.

Besonders vorteilhafte Stärkeether sind z. B. solche, die durch Veretherung von Stärke mit Tetramethylolacetylendiharnstoff erhältlich sind und welche als Amylum non mucilaginosum (nicht quellende Stärke) bezeichnet werden.

Insbesondere vorteilhaft sind ferner Stärkeester und deren Salze, beispielsweise die Natrium- und/oder Aluminiumsalze niedrigsubstituierter Halbester der Stärke, insbesondere Natrium Stärke n-Octenylsuccinat der Strukturformel (I), worin R sich durch die folgende Struktur auszeichnet 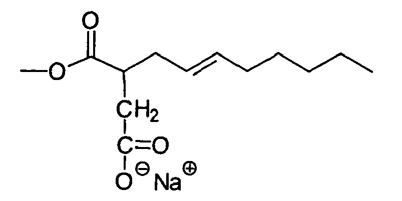 und welches z. B. unter der Handelsbezeichnung Amiogum® 23 bei der Firma CERESTAR erhältlich ist sowie Aluminium Stärke Octenylsuccinate, insbesondere die unter den Handelsbezeichnungen Dry Flo® Elite LL und Dry Flo® PC bei der Firma CERESTAR erhältlichen.

Vorteilhaft ist es, den mittleren Partikeldurchmesser der verwendeten modifizierten Polysaccharide kleiner als 20 µm, besonders vorteilhaft kleiner als 15 µm zu wählen.

Die Liste der genannten modifizierten Polysaccharide, die erfindungsgemäße Pickering-Emulsionen stabilisieren können, soll selbstverständlich nicht limitierend sein. Modifizierte Polysaccharide im Sinne der vorliegenden Erfindung sind auf zahlreichen, an sich bekannten Wegen, sowohl chemischer als auch physikalischer Natur erhältlich.

Vorteilhaft in allen vorgenannten Fällen ist es, die Konzentration der erfindungsgemäßen amphiphilen Pigmente größer als 1 Gew.-%, insbesondere zwischen 1 Gew.-% und 30 Gew.-%, besonders vorteilhaft zwischen 2,5 Gew.-% und 7,5 Gew.-%, bezogen auf das Gesamtgewicht der Zubereitungen, zu wählen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1:

**PIT - Emulsionen (Spray)**

| | |
|---|---|
| Glycerinmonostearat SE | 0,50 |
| PEG-100 Stearat | 5,00 |
| Cetyl Alkohol | 2,50 |
| Cetyl Dimethicon Copolyol | 0,50 |
| **DHA** | **2,0** |
| Hektorit | 0,50 |
| Butyl Methoxydibenzoylmethan | 1,50 |
| Ethylhexyl Methoxycinnamat | 8,00 |
| Ethylhexyl Salicylat | 4,00 |
| Phenylbenzmidazol Sulfonsäure | 1,00 |
| Dicaprylyl Ether | 4,00 |
| Phenyltrimethicon | 2,00 |
| Glycerin | 10,0 |
| Tocopherol | 1,00 |
| lodopropyl Butylcarbamt | 0,12 |
| Phenoxyethanol | 0,50 |
| Parfüm | 0,20 |
| Wasser | ad. 100 |

### Beispiel 2:

**PIT - Emulsionen (Spray)**

| | |
|---|---|
| Glycerinmonostearat SE | 2,00 |
| Isoceteth-20 | 0,50 |
| Ceteareth-12 | 5,00 |
| Cetyl Alkohol | 1,00 |
| **DHA** | **3,5** |
| Bentonit | 0,50 |
| Cocoglyceride | 3,00 |
| Butylenglycol Dicaprylat/Dicaprat | 4,00 |
| Cyclomethicon | 3,00 |
| Glycerin | 5,00 |
| Shea Butter | 2,00 |
| Methylparaben | 0,50 |
| Phenoxyethanol | 0,40 |
| Octoxyglycerin | 0,30 |
| Trisodium EDTA | 0,40 |
| Wasser | ad. 100 |

### Beispiel 3:

**PIT - Emulsionen (Spray)**

| | |
|---|---|
| Glycerinmonostearat SE | 3,00 |
| PEG-100 Stearat | 1,00 |
| **DHA** | **1,0** |
| Hektorit | 0,30 |
| Aniso Triazin | 0,50 |
| Butyl Methoxydibenzoylmethan | 1,00 |
| Terephthaliden Dicampher Sulfonsäure | 0,50 |
| Drometrizol Trisiloxan | 2,00 |
| Ethylhexyl Triazon | 2,00 |
| Octocrylen | 5,00 |
| Dibutyl Adipate | 2,50 |
| Methylparaben | 0,25 |
| Parfüm | 0,20 |
| Wasser | ad. 100 |

### Beispiel 4:

**PIT - Emulsionen (Spray)**

| | |
|---|---|
| Glycerinmonostearat SE | 5,00 |
| Ceteareth-12 | 1,00 |
| Ceteareth-20 | 2,00 |
| Cetyl Alkohol | 1,50 |
| Cetyl Palmitat | 0,50 |
| Polyglyceryl-2 Dipolyhydroxystearat | 0,75 |
| **DHA** | **1,0** |
| Stearalkoniumhektorit | 0,30 |
| Aniso Triazin | 2,00 |
| Ethylhexyl Methoxycinnamat | 4,50 |
| Ethylhexyl Salicylat | |
| Dioctyl Butamidotriazon | 3,00 |
| Ethylhexyl Triazon | 4,00 |
| Phenylbenzmidazol Sulfonsäure | 3,00 |
| Cocoglyceride | 3,00 |
| Butylenglycol Dicaprylat/Dicaprat | 3,00 |
| Dicaprylyl Carbonat | 0,50 |
| PVP Hexadecen Copolymer | 1,00 |
| Glycerin | 7,50 |
| Tocopherol | 0,75 |
| DMDM Hydantoin | 0,10 |
| Phenoxyethanol | 1,00 |
| Ethanol | 2,00 |
| Trisodium EDTA | 0,15 |
| Parfüm | 0,20 |
| Wasser | ad. 100 |

### Beispiel 5:

**PIT - Emulsionen (Spray)**

| | |
|---|---|
| Glyceryl Isostearat | 3,50 |
| Isoceteth-20 | 2,00 |
| PEG-100 Stearat | 1,00 |
| Cetyl Dimethicon Copolyol | 0,50 |
| Polyglyceryl-2 Dipolyhydroxystearat | 0,25 |
| **DHA** | **0,5** |
| Hektorit | 0,5 |
| Butyl Methoxydibenzoylmethan | 5,00 |
| Bisimidazylat | 1,00 |
| Ethylhexyl Methoxycinnamat | 5,00 |
| Ethylhexyl Salicylat | 3,50 |
| Dioctyl Butamidotriazon | 2,00 |
| Octocrylen | 8,00 |
| Phenylbenzmidazol Sulfonsäure | 1,00 |
| PVP Hexadecen Copolymer | 1,50 |
| Tocopherol | 0,50 |
| lodopropyl Butylcarbamt | 0,20 |
| Methylparaben | 0,45 |
| Octoxyglycerin | 1,00 |
| Parfüm | 0,45 |
| Wasser | ad. 100 |

### Beispiel 6:

**PIT - Emulsionen (Spray)**

| | |
|---|---|
| Glyceryl lsostearat | 4,00 |
| Ceteareth-20 | 2,50 |
| Cetyl Alkohol | 0,50 |
| Cetyl Palmitat | 1,00 |
| **DHA** | **4,00** |
| Henna | 1,00 |
| Bentonit | 0,30 |
| Hektorit | 0,30 |
| Aniso Triazin | 3,00 |
| Drometrizol Trisiloxan | 3,00 |
| Ethylhexyl Methoxycinnamat | 8,00 |
| Ethylhexyl Salicylat | 4,00 |
| Dioctyl Butamidotriazon | 2,00 |
| C12-15 Alkyl Benzoat | 4,00 |
| Cocoglyceride | 2,50 |
| Dibutyl Adipate | 3,00 |
| Phenyltrimethicon | 3,00 |
| lodopropyl Butylcarbamt | 0,15 |
| Trisodium EDTA | 0,20 |
| Wasser | ad. 100 |

### Beispiel 7:

**PIT - Emulsionen (Spray)**

| | |
|---|---|
| Glycerinmonostearat SE | 0,50 |
| Glyceryl Isostearat | 2,00 |
| Ceteareth-20 | 3,00 |
| Cetyl Alkohol | 1,50 |
| Cetyl Dimethicon Copolyol | 1,00 |
| **DHA** | **5,00** |
| Hektorit | 0,40 |
| Terephthaliden Dicampher Sulfonsäure | 1,00 |
| Ethylhexyl Triazon | 1,50 |
| Tocopherol | 1,00 |
| Ethanol | 7,50 |
| Wasser | ad. 100 |

### Beispiel 8:

**PIT - Emulsionen (Spray)**

| | |
|---|---|
| Glycerinmonostearat SE | 4,00 |
| Ceteareth-12 | 3,50 |
| PEG-100 Stearat | 0,50 |
| **DHA** | **1,00** |
| Stearalkoniumhektorit | 0,30 |
| Drometrizol Trisiloxan | 1,00 |
| Dioctyl Butamidotriazon | 1,50 |
| Ethylhexyl Triazon | 3,00 |
| Octocrylen | 7,50 |
| Al-Stearat + TiO₂ | 1,50 |
| Cocoglyceride | 3,50 |
| Dicaprylyl Carbonat | 6,00 |
| Dibutyl Adipate | 1,00 |
| Cyclomethicon | 4,00 |
| Shea Butter | 0,50 |
| Phenoxyethanol | 1,00 |
| Ethanol | 4,00 |
| Trisodium EDTA | 0,50 |
| Parfüm | 0,20 |
| Wasser | ad. 100 |

### Beispiel 9:

**O/W - Emulsions-Spray**

| | |
|---|---|
| Xanthan Gum | 0,7 |
| Ceteareth-20 | 1,0 |
| Polyglyceryl-2 Dipolyhydroxystearat | 0,2 |
| **DHA** | **0,5** |
| Hektorit | 0,5 |
| Bisimidazylat | 0,5 |
| Terephthaliden Dicampher Sulfonsäure | 0,5 |
| Drometrizol Trisiloxan | 3,0 |
| Octocrylen | 6,0 |
| Dioctyl Butamidotriazon | 1,0 |
| 4-Methylbenzyliden Campher | 2,0 |
| Titandioxid | 2,0 |
| C12-15 Alkyl Benzoat | 5,0 |
| Cocoglyceride | 2,5 |
| Dimethicon | 5,0 |
| Glycerin | 0,3 |
| Ascorbyl - Palmitat | 0,5 |
| Glycin Soja | 1,5 |
| Phenoxyethanol | 1,0 |
| Trisodium EDTA | 0,1 |
| Parfüm | 0,4 |
| Wasser | ad. 100 |

### Beispiel 10:

**Hydrodispersionsspray**

| | |
|---|---|
| Xanthan Gum | 0,2 |
| Bentonit | 0,5 |
| Hydroxypropyl Cellulose | 0,5 |
| **DHA** | **2,0** |
| Aniso Triazin | 2,0 |
| Ethylhexyl Methoxycinnamat | 10,0 |
| Ethylhexyl Salicylat | 5,0 |
| Homosalat | 4,0 |
| Octocrylen | 4,0 |
| Dioctyl Butamidotriazon | 2,5 |
| Phenylbenzmidazol Sulfonsäure | 1,0 |
| PVP Hexadecen Copolymer | 0,75 |
| Glycerin | 10,0 |
| Vitamin E -Acetat | 0,5 |
| DMDM Hydantoin | 0,12 |
| Propylparaben | 0,02 |
| Trisodium EDTA | 0,3 |
| Wasser | ad. 100 |

### Beispiel 11:

**O/W - Emulsions-Spray**

| | |
|---|---|
| Acrylates/C10-30 Alky Acrylate Crosspolymer | 0,3 |
| Hektorit | 0,2 |
| PEG-40 Stearat | 2,0 |
| Cetyl Alkohol | 0,5 |
| **DHA** | **3,5** |
| Butyl Methoxydibenzoylmethan | 0,5 |
| Octocrylen | 2,0 |
| Dimethicon Diethylbenzalmalonat | 4,0 |
| Ethylhexyl Triazon | 3,0 |
| Butylenglycol Dicaprylat/Dicaprat | 7,5 |
| Dicaprylyl Carbonat | 4,0 |
| Dimethicon | 2,5 |
| Glycerin | 7,5 |
| Vitamin E -Acetat | 0,75 |
| Ascorbyl - Palmitat | 0,25 |
| Vaseline | 2,0 |
| Phenoxyethanol | 1,0 |
| Ethanol | 3,0 |
| Farbstoff wasserlöslich | 0,02 |
| Parfüm | 0,2 |
| Wasser | ad. 100 |

### Beispiel 12:

**Hydrodispersionsspray**

| | |
|---|---|
| Hektorit | 0,4 |
| Xanthan Gum | 0,2 |
| Hydroxypropyl Cellulose | 1,0 |
| **DHA** | **1,5** |
| Aniso Triazin | 3,0 |
| Bisimidazylat | 0,5 |
| Drometrizol Trisiloxan | 1,0 |
| Ethylhexyl Methoxycinnamat | 7,5 |
| Ethylhexyl Salicylat | 0,5 |
| Octocrylen | 7,5 |
| Ethylhexyl Triazon | 2,0 |
| Phenylbenzmidazol Sulfonsäure | 2,0 |
| Titandioxid | 3,0 |
| Glycerin | 5,0 |
| Glycin Soja | 2,0 |
| Ethanol | 7,5 |
| Trisodium EDTA | 0,5 |
| Wasser | ad. 100 |

### Beispiel 13:

**O/W - Emulsionsspray**

| | |
|---|---|
| Bentonit | 1,0 |
| Ceteareth-20 | 2,0 |
| Cetyl Alkohol | 1,0 |
| Hydrögenated Cocoglyceride | 0,5 |
| **DHA** | **0,5** |
| Aniso Triazin | 2,0 |
| Butyl Methoxydibenzoylmethan | 1,0 |
| Terephthaliden Dicampher Sulfonsäure | 0,5 |
| Zinkoxid | 3,0 |
| C12-15 Alkyl Benzoat | 5,0 |
| Dicaprylyl Ether | 5,0 |
| Dimethicon | 1,0 |
| PVP Hexadecen Copolymer | 0,5 |
| Vitamin E -Acetat | 1,0 |
| lodopropyl Butylcarbamat | 0,18 |
| Phenoxyethanol | 0,75 |
| Parfüm | 0,3 |
| Wasser | ad. 100 |

### Beispiel 14:

**O/W - Emulsions-Spray**

| | |
|---|---|
| Hydroxypropyl Cellulose | 0,5 |
| Hektorit | 0,3 |
| PEG-40 Stearat | 1,0 |
| Hydrogenated Cocoglyceride | 1,0 |
| Polyglyceryl-2 Dipolyhydroxystearat | 0,5 |
| **DHA** | **2,5** |
| Bisimidazylat | 3,0 |
| Ethylhexyl Salicylat | 1,0 |
| Dimethicon Diethylbenzalmalonat | 6,0 |
| Ethylhexyl Triazon | 1,5 |
| 4-Methylbenzyliden Campher | 2,0 |
| Titandioxid | 0,5 |
| Zinkoxid | 1,0 |
| Paraffinöl | 7,0 |
| PVP Hexadecen Copolymer | 1,0 |
| Glycerin | 5,0 |
| Vaseline | 1,0 |
| Ethanol | 5,0 |
| Farbstoff wasserlöslich | 0,10 |
| Parfüm | 0,3 |
| Wasser | ad. 100 |

### Beispiel 15:

**Hydrodispersionsspray**

| | |
|---|---|
| Xanthan Gum | 1,0 |
| Stearalkoniumhektorit | 0,5 |
| **DHA** | **3,0** |
| Aniso Triazin | 0,5 |
| Butyl Methoxydibenzoylmethan | 0,5 |
| Bisimidazylat | 0,5 |
| Terephthaliden Dicampher Sulfonsäure | 0,5 |
| Dioctyl Butamidotriazon | 3,5 |
| Titandioxid | 4,0 |
| Cocoglyceride | 8,0 |
| Dicaprylyl Carbonat | 10,0 |
| PVP Hexadecen Copolymer | 1,0 |
| Vitamin E -Acetat | 1,5 |
| lodopropyl Butylcarbamat | 0,2 |
| Propylparaben | 0,1 |
| Trisodium EDTA | 0,2 |
| Parfüm | 0,1 |
| Wasser | ad. 100 |

### Beispiel 16:

**Hydrodispersionsspray**

| | |
|---|---|
| Hektorit | 1,5 |
| **Henna** | **4,0** |
| Drometrizol Trisiloxan | 4,0 |
| Ethylhexyl Methoxycinnamat | 5,0 |
| Octocrylen | 10,0 |
| Ethylhexyl Triazon | 4,0 |
| Titandioxid | 1,5 |
| Cocoglyceride | 4,0 |
| Butylenglycol Dicaprylat/Dicaprat | 6,0 |
| Glycerin | 7,5 |
| Ascorbyl - Palmitat | 0,75 |
| DMDM Hydantoin | 0,2 |
| Ethanol | 3,5 |
| Trisodium EDTA | 0,15 |
| Wasser | ad. 100 |

### Beispiel 17:

**O/W Pickering-Creme**

| | |
|---|---|
| Octyldodecanol | 3,00 |
| Caprylic/Capric Triglycerid | 3,00 |
| C12-15- Alkyl Benzoat | 3,00 |
| PVP/Hexadecene Copolymer | 0,50 |
| Hektorit | 1,00 |
| Xanthan Gum | 0,10 |
| Glyceryl Stearate Citrat | 1,00 |
| Cyclomethicon | 2,00 |
| Vitamin E-Acetat | 0,50 |
| Ethylhexylmethoxycinnamat | 7,50 |
| Butyl Methoxydibenzoylmethan | 2,00 |
| Bis-Ethylhexyloxyphenol Methoxyphenyl Triazin | 1,00 |
| Titandioxid + Alumina + Simethicon + Aqua | 0,60 |
| Phenyl Dibenzimidazol Tetrasulfonic Acid | 0,5% |
| Distarch Phosphat | 6,00 |
| Silica | 0,60 |
| Citronensäure | 0,086 |
| Sodium Citrat | 0,174 |
| DHA | 2,00 |
| Glycerin | 4,00 |
| Butyleneglycol | 4,00 |
| Octoxyglycerin | 0,30 |
| Trisodium EDTA | 1,00 |
| Glucosylrutin | 0,50 |
| Methylparaben | 0,20 |
| Phenoxyethanol | 0,30 |
| lodopropynyl Butylcarbamat | 0,18 |
| Ethanol | 5,00 |
| Parfüm | 0,30 |
| Wasser | Ad 100 |

### Beispiel 18:

**O/W Pickering-Creme**

| | |
|---|---|
| Octyldodecanol | 19,00 |
| C12-15- Alkyl Benzoat | 19,00 |
| Dicaprylyl Ether | 8,00 |
| Hydrogenated Polyisobutene | 7,00 |
| Disteardimonium Hectorite | 0,50 |
| Xanthan Gum | 0,20 |
| Phenyltrimethicon | 2,00 |
| Vitamin E-Acetat | 1,00 |
| Octocrylen | 5,00 |
| Isopropyl Dibenzoyl Methan | 0,50 |
| Titandioxid + Alumina + Simethicon + Aqua | 1,50 |
| Corn Starch Modified | 1,00 |
| Glycerin | 7,50 |
| Trisodium EDTA | 0,50 |
| Ubiquinone | 0,25 |
| Phenoxyethanol | 0,50 |
| Diazolidinyl Urea | 0,28 |
| Wasser | Ad 100 |

### Beispiel 19:

**Pickering-Lotion**

| | |
|---|---|
| Mineralöl | 16,00 |
| Octyldodecanol | 5,00 |
| Caprylic/Capric Triglycerid | 6,00 |
| Hydroxyoctacosanyl Hydroxystearat | 1,50 |
| PVP/Hexadecene Copolymer | 1,00 |
| Disteardimonium Hectorite | 1,00 |
| Cyclomethicon | 2,00 |
| Butyl Methoxydibenzoylmethan | 0,50 |
| Ethylhexyltriazon | 1,50 |
| Titandioxid + Alumina + Simethicon + Aqua | 2,00 |
| Zinkoxid | 2,00 |
| Silica Dimethyl Silylat | 0,50 |
| Magnesium Sulfat | 0,70 |
| Glycerin | 3,00 |
| DHA | 3,00 |
| Butylenglycol | 5,00 |
| Trisodium EDTA | 1,00 |
| Porpylene Carbonat | 0,33 |
| Methylparaben | 0,21 |
| Propylparaben | 0,07 |
| Phenoxyethanol | 0,50 |
| Wasser | Ad 100 |

### Beispiel 20:

**O/W Pickering-Emulsion**

| | |
|---|---|
| Titandioxid (Eusolex T2000) | 3 |
| Titandioxid (Titandioxid T805) | 3 |
| Talkum (Talkum Micron) | 2 |
| Tapiokastärke | 4 |
| Hydrogenierte Coco Glyceride | 1 |
| Octyldodecanol | 4,25 |
| Mineralöl | 3,65 |
| Butylen Glycol Caprylat/Caprat | 5 |
| PVP/ Hecadecene Copolymer | 0,5 |
| Stearyl Alcohol | 1,5 |
| Octocrylene | 3,5 |
| Cyclomethicon | 2 |
| Tocopheryl Acetat | 0,5 |
| PEG-40 Hydrogenated Castor Oil | 1 |
| Ethylhexyl Methoxycinnamät | 5 |
| Bis- Ethylhexyloxyphenol Methoxyphenyl Triazin | 1,7 |
| Konservierung | 1 |
| Glycerin | 7 |
| DHA | 1 |
| Hektorit | 3 |
| Milchsäure | 0,3 |
| Hydroxyethylcellulose | 0,5 |
| Methylhydroxyethylcellulose | 0,1 |
| EDTA-Lösung | 1 |
| Parfum | 0,4 |
| Wasser | ad 100 |

### Beispiel 21:

**O/W Pickering-Emulsion**

| | |
|---|---|
| Titandioxid (Eusolex T2000) | 2,5 |
| Zinkoxid | 4 |
| Di Stärkephosphat | 1,5 |
| Hydrogenierte Coco Glyceride | 1 |
| C_{16 - 38} Alkylhydroxystearoyl-stearat (Kesterwachs K80P) | 0,5 |
| Cetyl Dimethicon (Abil Wax 9840) | 0,5 |
| Octyldodecanol | 3,75 |
| Butylen Glycol Caprylat/Caprat | 5 |
| PVP/ Hecadecene Copolymer | 0,5 |
| Stearyl Alcohol | 1,5 |
| Octocrylene | 3,5 |
| Cyclomethicon | 2 |
| Tocopheryl Acetat | 0,5 |
| PPG-15 Stearyl Ether | 1 |
| Dicaprylyl Ether (Cetiol OE) | 3,5 |
| Ethylhexyl Methoxycinnamat | 5 |
| Bis- Ethylhexyloxyphenol Methoxyphenyl Triazin | 2 |
| Phenyl Dibenzimidazol Tetrasulfonsäure | 2 |
| Konservierung | 1 |
| Glycerin | 5 |
| DHA | 5 |
| Hektorit | 0,5 |
| Milchsäure | 1 |
| MgSO₄ | 0,9 |
| Hydroxyethylcellulose | 0,7 |
| NaOH 45%ige Lösung in Wasser | 0,5 |
| EDTA-Lösung | 1 |
| Parfum | 0,4 |
| Wasser | ad 100 |

### Beispiel 22:

**O/W Pickering-Emulsion**

| | |
|---|---|
| Titandioxid (Eusolex T2000) | 2 |
| Talkum (Talkum Micron) | 1 |
| Di Stärkephosphat | 1 |
| Tapiokastärke | 3 |
| C₂₀₋₄₀ Alkylstearat (Kesterwachs K82) | 0,5 |
| Behenoxy Dimethicon (Abil Wax 2440) | 1 |
| Polyisobuten (Rewopal PIB 1000) | 0,5 |
| Caprylic/Capric Triglycerid | 5 |
| Octyldodecanol | 5 |
| C₁₂₋₁₅ Alkyl Benzoat | 5 |
| Acetylated Glycol Stearate + Tristearin | 2 |
| Stearyl Alcohol | 1 |
| Octocrylene | 3 |
| Dimethicon | 2 |
| Tocopheryl Acetat | 1 |
| Starch Hydroxypropyltrimonium Chlorid (Sensomer CI 50) | 1 |
| Ethylhexyl Methoxycinnamat | 5 |
| Bis- Ethylhexyloxyphenol Methoxyphenyl Triazin | 1,5 |
| Glycerin | 5 |
| DHA | 3 |
| Bentonit | 0,5 |
| Biosaccharid Gel (Fucogel 1000) | 0,5 |
| Polyquaternium 37 | 0,2 |
| EDTA-Lösung | 1 |
| Alkohol | 15 |
| Parfum | 0,3 |
| Wasser | ad 100 |

### Beispiel 23:

**O/W Pickering-Emulsion**

| | |
|---|---|
| Titandioxid (Eusolex T2000) | 2 |
| Zinkoxid | 4 |
| Natrium Maisstärke n-Octenylsuccinat | 1 |
| Di Stärkephosphat | 1 |
| Tapiokastärke | 5 |
| Hydrogenierte Coco Glyceride | 1 |
| Behenoxy Dimethicon (Abil Wax 2440) | 1 |
| Caprylic/Capric Triglycerid | 4 |
| Octyldodecanol | 4 |
| Mineralöl | 4 |
| Stearyl Alcohol | 1 |
| Cyclomethicon | 3 |
| Konservierung | 1 |
| Glycerin | 3 |
| DHA | 5 |
| Hektorit | 0,3 |
| Bentonit | 0,5 |
| Milchsäure | 0,8 |
| Hydroxypropyl Methylcellulose | 0,1 |
| Polyquaternium 37 | 0,2 |
| Wasser | ad 100 |

### Beispiel 24:

**O/W Pickering-Emulsion**

| | |
|---|---|
| Titandioxid (Eusolex T2000) | 0,5 |
| Zinkoxid | 0,5 |
| Silica (Aerosil R972) | 0,5 |
| Mineralöl | 8 |
| Konservierung | 1 |
| Glycerin | 1 |
| DHA | 2 |
| Henna | 2 |
| Hektorit | 1 |
| Citronensäure | 0,08 |
| Natrium Citrat | 0,2 |
| Kalium Sorbat | 0,1 |
| Hydroxyethylcellulose | 0,6 |
| EDTA-Lösung | 0,6 |
| Parfum | 0,5 |
| Wasser | ad 100 |

### Beispiel 25:

**O/W Pickering-Emulsion**

| | |
|---|---|
| Titandioxid (Eusolex T2000) | 3 |
| Natrium Maisstärke n-Octenylsuccinat | 0,5 |
| C_{16 - 38} Alkylhydroxystearoylstearat (Kesterwachs K80P) | 3 |
| Caprylic/Capric Triglycerid | 8 |
| Octyldodecanol | 8 |
| Mineralöl | 8 |
| C₁₂₋₁₅ Alkyl Benzoat | 5 |
| Dicaprylyl Ether (Cetiol OE) | 5 |
| Ethylhexyltriazon | 4 |
| Butyl Methoxydibenzoylmethan | 2 |
| Phenyl Dibenzimidazol Tetrasulfonsäure | 2 |
| Glycerin | 5 |
| DHA | 0,5 |
| Hektorit | 1,5 |
| NaCl | 0,9 |
| Xanthan Gummi | 0,1 |
| NaOH 45%ige Lösung in Wasser | 1,2 |
| EDTA-Lösung | 1 |
| Alkohol | 10 |
| Wasser | ad 100 |

### Beispiel 26:

**O/W Pickering-Emulsion**

| | |
|---|---|
| Titandioxid (Eusolex T2000) | 2 |
| Titandioxid (Titandioxid T805) | 3 |
| Silica (Aerosil R972) | 0,5 |
| Talkum (Talkum Micron) | 2 |
| Bornitrid | 1 |
| Cetyl Dimethicon (Abil Wax 9840) | 1 |
| Caprylic/Capric Triglycerid | 5 |
| Butylen Glycol Caprylat/Caprat | 5 |
| C₁₂₋₁₅ Alkyl Benzoat | 3 |
| PVP/ Hecadecene Copolymer | 1 |
| Acetylated Glycol Stearate + Tristearin | 0,5 |
| Dimethicon | 2 |
| Tocopheryl Acetat | 1 |
| Dicaprylyl Ether (Cetiol OE) | 3 |
| Ethylhexyl Methoxycinnamat | 4 |
| Bis- Ethylhexyloxyphenol Methoxyphenyl Triazin | 1 |
| Octocrylen | 2 |
| Butyl Methoxydibenzoylmethan | 2 |
| Diethylhexyl Butamido Triazon (UVASORB HEB) | 1 |
| Phenyl Dibenzimidazol Tetrasulfonsäure | 2 |
| Phenylbenzimidazol Sulfonsäure | 2 |
| Henna | 4 |
| Bentonit | 1 |
| NaOH 45%ige Lösung in Wasser | 2 |
| Alkohol | 15 |
| Wasser | ad 100 |

### Beispiel 27:

**O/W Sonnenschutz Emulsionen**

| | |
|---|---|
| Glycerinmonostearat SE | 0,50 |
| Glyceryl Stearat Citrat | 2,00 |
| PEG-40 Stearat | 0,50 |
| Cetyl Alkohol | 2,50 |
| Disodium Phenyl Dibenzimidazol Tetrasulfonat | 2,50 |
| Ethylhexyl Triazon | 2,00 |
| Methylen Bis-Benztriazolyl Tetramethylbutylphenol | 2,00 |
| Titandioxid MT-100Z | 1,00 |
| Butylenglycol Dicaprylat/Dicaprat | 5,00 |
| Cyclomethicon | 2,00 |
| PVP Hexadecen Copolymer | 0,50 |
| Glycerin | 3,00 |
| Xanthan Gummi | 0,15 |
| Hektorit | 0,50 |
| DHA | 1,00 |
| Henna | 2,00 |
| Vitamin E Acetat | 0,50 |
| Alpha-Glucosylrutin | 0,25 |
| Methylparaben | 0,15 |
| Phenoxyethanol | 1,00 |
| Parfüm | 0,20 |
| Wasser | ad 100 |

### Beispiel 28:

**O/W Sonnenschutz Emulsionen**

| | |
|---|---|
| Glycerinmonostearat SE | 1,00 |
| Stearinsäure | 3,00 |
| Cetyl Alkohol | 1,00 |
| UVASorb® K2A | 2,50 |
| Phenylbenzmidazol Sulfonsäure | 0,50 |
| Disodium Phenyl Dibenzimidazol Tetrasulfonat | 1,00 |
| Uvinul® A Plus | 1,00 |
| Bis-Ethylhexyloxyphenol Methoxyphenyl Triazin | 1,50 |
| Diethylhexyl Butamido Triazon | 2,00 |
| Ethylhexyl Methoxycinnamat | 3,50 |
| Octocrylen | 5,00 |
| Homosalat | 2,00 |
| Titandioxid T 805 | 1,50 |
| C12-15 Alkyl Benzoat | 2,50 |
| Dimethicon | 0,50 |
| Shea Butter | 2,00 |
| Glycerin | 7,50 |
| Henna | 3,50 |
| Bentonit | 1,00 |
| DMDM Hydantoin | 0,60 |
| Phenoxyethanol | 0,40 |
| EDTA | 0,20 |
| Ethanol | 2,00 |
| Parfüm | 0,20 |
| Wasser | ad 100 |

### Beispiel 29:

**O/W Sonnenschutz Emulsionen**

| | |
|---|---|
| Glycerinmonostearat SE | 3,00 |
| Stearinsäure | 0,75 |
| PEG-100 Stearat | 1,50 |
| Cetyl Phosphat | 0,75 |
| Stearyl Alkohol | 3,00 |
| Mineralöl | 9,00 |
| Caprylic Capric Triglycerid | 3,00 |
| Dicaprylyl Ether | 3,50 |
| Dibutyl Adipat | 6,00 |
| Dimethicon | 1,00 |
| Xanthan Gummi | 0,05 |
| Hektorit | 1,00 |
| Vitamin E Acetat | 0,25 |
| Fucogel® 1000 | 1,50 |
| DHA | 2,50 |
| DMDM Hydantoin | 0,40 |
| Methylparaben | 0,25 |
| EDTA | 0,35 |
| Ethanol | 1,50 |
| Wasser | ad 100 |

### Beispiel 30:

**O/W Sonnenschutz Emulsionen**

| | |
|---|---|
| Glyceryl Stearat Citrat | 1,00 |
| Stearinsäure | 2,00 |
| Cetyl Dimethicon Copolyol | 0,75 |
| Disodium Phenyl Dibenzimidazol Tetrasulfonat | 2,00 |
| Terephthaliden Dicamphor Sulfonsäure | 0,50 |
| Ethylhexyl Triazon | 2,00 |
| Ethylhexyl Methoxycinnamat | 10,00 |
| Octocrylen | 7,50 |
| Titandioxid MT-100Z | 3,00 |
| Cyclomethicon | 4,50 |
| PVP Hexadecen Copolymer | 0,50 |
| Glycerin | 7,50 |
| DHA | 0,50 |
| Stearalkoliumhektorit | 0,20 |
| Vitamin E Acetat | 0,50 |
| Alpha-Glucosylrutin | 0,20 |
| DMDM Hydantoin | 0,20 |
| Konkaben LMB ® | 0,18 |
| Phenoxyethanol | 0,40 |
| EDTA | 0,50 |
| Wasser | ad 100 |

### Beispiel 31:

**O/W Sonnenschutz Emulsionen**

| | |
|---|---|
| Glyceryl Stearat Citrat | 2,00 |
| Cetyl Phosphat | 1,00 |
| Cetyl Alkohol | 0,50 |
| Phenylbenzmidazol Sulfonsäure | 1,00 |
| Disodium Phenyl Dibenzimidazol Tetrasulfonat | 1,50 |
| Butyl Methoxydibenzoylmethan | 2,00 |
| Ethylhexyl Triazon | 4,00 |
| Titandioxid T 805 | 1,00 |
| Titandioxid MT-100Z | 1,00 |
| Dicaprylyl Ether | 2,00 |
| Dimethicon | 2,00 |
| PVP Hexadecen Copolymer | 1,00 |
| DHA | 3,00 |
| Hektorit | 1,00 |
| Glycerin | 5,00 |
| Vitamin E Acetat | 0,75 |
| Konkaben LMB ® | 0,20 |
| Methylparaben | 0,50 |
| Phenoxyethanol | 0,50 |
| EDTA | 0,02 |
| Ethanol | 3,00 |
| Wasser | ad 100 |

### Beispiel 32:

**O/W Sonnenschutz Emulsionen**

| | |
|---|---|
| Glycerinmonostearat SE | 1,50 |
| PEG-40 Stearat | 2,00 |
| Cetyl Dimethicon Copolyol | 0,50 |
| Stearyl Alkohol | 2,00 |
| Phenylbenzmidazol Sulfonsäure | 2,00 |
| Ethylhexyl Triazon | 2,00 |
| Drometrizol Trisiloxan | 1,00 |
| Titandioxid T 805 | 0,50 |
| C12-15 Alkyl Benzoat | 7,00 |
| Dibutyl Adipat | 2,00 |
| Alpha-Glucosylrutin | 0,25 |
| Fucogel® 1000 | 5,00 |
| Bentonit | 0,50 |
| DHA | 1,50 |
| Ethanol | 5,00 |
| Parfüm | 0,30 |
| Wasser | ad 100 |

### Beispiel 33:

**O/W Sonnenschutz Emulsionen**

| | |
|---|---|
| Glyceryl Stearat Citrat | 2,50 |
| Stearyl Alkohol | 0,50 |
| Cetyl Alkohol | 2,00 |
| Phenylbenzmidazol Sulfonsäure | 0,50 |
| Disodium Phenyl Dibenzimidazol Tetrasulfonat | 1,00 |
| Terephthaliden Dicamphor Sulfonsäure | 0,50 |
| Butyl Methoxydibenzoylmethan | 1,00 |
| Bis-Ethylhexyloxyphenol Methoxyphenyl Triazin | 2,50 |
| Octocrylen | 2,50 |
| Ethylhexylsalicylat | 5,00 |
| Zinkoxid NDM | 3,00 |
| C12-15 Alkyl Benzoat | 5,00 |
| Dibutyl Adipat | 2,00 |
| Cyclomethicon | 0,50 |
| Shea Butter | 0,50 |
| PVP Hexadecen Copolymer | 1,00 |
| Glycerin | 2,50 |
| Xanthan Gummi | 0,30 |
| Hektorit | 1,50 |
| Vitamin E Acetat | 1,00 |
| Konkaben LMB ® | 0,15 |
| Phenoxyethanol | 0,60 |
| DHA | 0,50 |
| EDTA | 0,03 |
| Ethanol | 1,00 |
| Parfüm | 0,40 |
| Wasser | ad 100 |

### Beispiel 34:

**Hydrodispersion**

| | |
|---|---|
| Hydroxypropyl Cellulose | 1,0 |
| Xanthan Gummi | 1,0 |
| **Hektorit** | **1,5** |
| **AmDHA** | **5,0** |
| Aniso Triazin | 2,0 |
| Bisoctyltriazol | 6,0 |
| Phenylbenzmidazole Sulfonsäure | 1,0 |
| Bisimidazylate | 1,0 |
| Terephthalylidene Dicamphor Sulfonic Acid | 0,2 |
| Ethylhexyl Methoxycinnamat | 5,0 |
| Zinkoxid | 4,0 |
| Butylenglycol Dicaprylat/Dicaprat | 6,0 |
| PVP Hexadecen Copolymer | 0,5 |
| Glycerin | 7,5 |
| Trisodium EDTA | 1,0 |
| Zitronensäure | 0,1 |
| DMDM Hydantoin | 0,2 |
| Methylparaben | 0,15 |
| Phenoxyethanol | 1,0 |
| Parfüm | 0,2 |
| Wasser | ad. 100 |

### Beispiel 35:

**Hydrodispersion**

| | |
|---|---|
| Hydroxypropyl Cellulose | 1,50 |
| Xanthan Gummi | 1,0 |
| **Hektorit** | **2,0** |
| **AmDHA** | **1,5** |
| Caprylic/Capric Triglycerid | 2,5 |
| Hydrogenierte Coco-Glyceride | 5,0 |
| Glycerin | 2,5 |
| Tocopherol | 0,2 |
| Trisodium EDTA | 1,5 |
| Zitronensäure | 0,1 |
| Phenoxyethanol | 0,60 |
| Ethanol | 1,0 |
| Parfüm | 0,2 |
| Wasser | ad. 100 |

### Beispiel 36:

| | |
|---|---|
| SoHektorit | 0,5 |
| AcBentonit | 1,0 |
| **DHA** | **4,0** |
| Dioctyl Butamidotriazon | 2,0 |
| Aniso Triazin | 1,0 |
| Phenylbenzmidazole Sulfonsäure | 2,0 |
| Octocrylen | 10,0 |
| Butyl Methoxydibenzoylmethan | 1,0 |
| Titandioxid | 1,0 |
| C12-15 Alkyl Benzoat | 2,0 |
| Isohexadecen | 4,0 |
| Dibutyl Adipat | 2,0 |
| PVP Hexadecen Copolymer | 0,5 |
| Butylen Glycol | 3,0 |
| Trisodium EDTA | 1,0 |
| Natronlauge | 1,0 |
| Ethanol | 5,0 |
| Parfüm | 0,2 |
| Wasser | ad. 100 |

### Beispiel 37:

| | |
|---|---|
| SoHektorit | 1,5 |
| Xanthan Gummi | 0,6 |
| **DHA** | **0,5** |
| Dioctyl Butamidotriazon | 2,0 |
| Ethylhexyl Triazon | 4,0 |
| Drometrizole Trisiloxane | 3,0 |
| Ethylhexyl Methoxycinnamat | 10,0 |
| Butyl Methoxydibenzoylmethan | 1,0 |
| Titandioxid | 4,0 |
| C12-15 Alkyl Benzoat | 2,5 |
| Dicaprylyl Ether | 4,0 |
| Dicaprylyl Carbonat | 2,0 |
| Dibutyl Adipat | 0,5 |
| Jojobaöl | 2,0 |
| Butylen Glycol | 7,5 |
| Ascorbyl - Palmitat | 0,5 |
| Octoxyglycerin | 1,0 |
| Trisodium EDTA | 0,5 |
| Natronlauge | 0,2 |
| lodopropyl Butylcarbamat | 0,6 |
| Phenoxyethanol | 0,4 |
| Ethanol | 2,0 |
| Wasser | ad. 100 |

### Beispiel 38:

| | |
|---|---|
| SoHektorit | 1,0 |
| Hydroxypropyl Cellulose | 0,4 |
| Xanthan Gummi | 0,2 |
| **DHA** | **3,0** |
| Caprylic/Capric Triglycerid | 2,0 |
| C12-15 Alkyl Benzoat | 3,0 |
| Isohexadecen | 2,0 |
| Dibutyl Adipat | 1,0 |
| PVP Hexadecen Copolymer | 0,05 |
| Ascorbyl - Palmitat | 0,75 |
| Glycin Soja | 2,0 |
| Trisodium EDTA | 0,5 |
| Natronlauge | 0,25 |
| Ethanol | 7,0 |
| Wasser | ad. 100 |

### Beispiel 39:

| | |
|---|---|
| AcBentonit | 0,75 |
| Hydroxypropyl Cellulose | 1,0 |
| Xanthan Gummi | 1,0 |
| **DHA** | **2,0** |
| Dioctyl Butamidotriazon | 2,0 |
| Ethylhexyl Triazon | 4,0 |
| Aniso Triazin | 1,0 |
| Phenylbenzmidazole Sulfonsäure | 1,0 |
| Terephthalylidene Dicamphor Sulfonic Acid | 0,2 |
| Ethylhexyl Methoxycinnamat | 5,0 |
| Dimethicone-diethylbenzalmalonate | 4,0 |
| Homosalate | 5,0 |
| Zinkoxid | 4,0 |
| Paraffinöl | 1,0 |
| Isohexadecen | 6,0 |
| Cylomethicon | 3,0 |
| PVP Hexadecen Copolymer | 0,5 |
| Butylen Glycol | 7,5 |
| Octoxyglycerin | 0,5 |
| lodopropyl Butylcarbamat | 0,2 |
| Phenoxyethanol | 1,0 |
| Parfüm | 0,2 |
| Wasser | ad. 100 |

### Beispiel 40:

| | |
|---|---|
| AcBentonit | 1,0 |
| Xanthan Gummi | 1,0 |
| **DHA** | **4,0** |
| Butylen Glycol | 2,5 |
| Ascorbyl - Palmitat | 0,2 |
| Trisodium EDTA | 1,5 |
| Parfüm | 0,2 |
| Wasser | ad. 100 |

### Beispiel 41:

| | |
|---|---|
| SoHektorit | 0,5 |
| Hydroxypropyl Cellulose | 1,0 |
| **DHA** | **1,5** |
| Dioctyl Butamidotriazon | 1,0 |
| Aniso Triazin | 1,0 |
| Bisimidazylate | 3,5 |
| Terephthalylidene Dicamphor Sulfonic Acid | 1,0 |
| Ethylhexyl Salicylate | 5,0 |
| Butyl Methoxydibenzoylmethan | 0,5 |
| Titandioxid | 1,5 |
| PVP Hexadecen Copolymer | 0,5 |
| Butylen Glycol | 5,0 |
| Ascorbyl - Palmitat | 0,3 |
| Octoxyglycerin | 1,0 |
| Glycin Soja | 1,5 |
| Trisodium EDTA | 0,5 |
| Wasser | ad. 100 |

## Patentansprüche

1. Verwendung von mindestens einem Schichtsilikat oder einer Kombination von mindestens einem Schichtsilikat und Xanthan Gum zur Stabilisierung von wasserhaltigen kosmetischen Zubereitungen, die ein oder mehrere selbstbräunende Substanzen und und gegebenenfalls weitere kosmetischen und/oder dermatologischen Wirk-, Hilfs- und Zusatzstoffe enthalten, wobei es sich um O/W-Emulsionen, Hydrodispersionen, Pickering-Emulsionen oder Hydrogele handelt.

2. Verwendung nach Anspruch 1, **dadurch gekennzeichnet, dass** sie eine oder mehrere selbstbräunende Substanzen in einer Konzentration von 0,1 bis 10 Gew.-% und besonders bevorzugt von 0,5 bis 6 Gew.-%, jeweils bezogen auf das Gesamtgewicht der Zubereitung enthalten.

3. Verwendung nach mindestens einem der vorhergehenden Ansprüche, **dadurch gekennzeichnet, dass** sie ein oder mehrere modifizierte Schichtsilikate und/oder Tonmineralien in einer Summenkonzentration von 0,05 bis 10,0 Gew.-%, bevorzugt von 0,1 bis 5 Gew.-%, besonders bevorzugt von 1,0 bis 3,0 Gew.-% liegt, jeweils bezogen auf das Gesamtgewicht der Zubereitung enthalten.

4. Verwendung nach mindestens einem der vorhergehenden Ansprüche, **dadurch gekennzeichnet, dass** sie einen Emulgatoranteil von weniger als 1 Gew.-% enthalten, bezogen auf das Gesamtgewicht der Zubereitungen.

5. Verwendung nach mindestens einem der vorhergehenden Ansprüche, **dadurch gekennzeichnet, dass** sie im wesentlichen emulgatorfrei sind.

6. Verwendung nach mindestens einem der vorhergehenden Ansprüche, **dadurch gekennzeichnet, dass** sie frei von Carbomeren ist.

7. Verwendung nach mindestens einem der vorhergehenden Ansprüche, **dadurch gekennzeichnet, dass** sie als selbstbräunende Substanz 1,3-Dihydroxyaceton enthalten.

8. Verwendung nach mindestens einem der vorhergehenden Ansprüche, **dadurch gekennzeichnet, dass** sie organische und/oder anorganische Sonnenschutzfilter enthalten.

9. Verwendung nach mindestens einem der vorhergehenden Ansprüche, **dadurch gekennzeichnet, dass** sie anorganische und/oder organische und/oder modifizierte anorganische Pigmente enthalten.

10. Verwendung einer stabilisierten kosmetische Zubereitung nach mindestens einem der vorhergehenden Ansprüche zur Färbung der Haut von mehrzelligen Organismen, insbesondere der Haut von Mensch und Tier.

11. Verwendung einer stabilisierten kosmetische Zubereitung nach Anspruch 10 zur Farbangleichung von unterschiedlich pigmentierten Hautstellen.

## Claims

1. Use of at least one layered silicate or a combination of at least one layered silicate and xanthan gum for the stabilization of hydrous cosmetic preparations which comprise one or more self-tanning substances and optionally further cosmetic and/or dermatological active ingredients, auxiliaries and additives, which are O/W emulsions, hydrodispersions, Pickering emulsions or hydrogels.

2. Use according to Claim 1, **characterized in that** they comprise one or more self-tanning substances in a concentration of from 0.1 to 10 % by weight and particularly preferably from 0.5 to 6 % by weight, in each case based on the total weight of the preparation.

3. Use according to at least one of the preceding claims, **characterized in that** they comprise one or more modified layered silicates and/or clay minerals in a sum concentration of from 0.05 to 10.0 % by weight, preferably from 0.1 to 5 % by weight, particularly preferably from 1.0 to 3.0 % by weight, in each case based on the total weight of the preparation.

4. Use according to at least one of the preceding claims, **characterized in that** they comprise an emulsifier fraction of less than 1 % by weight, based on the total weight of the preparations.

5. Use according to at least one of the preceding claims, **characterized in that** they are essentially emulsifier-free.

6. Use according to at least one of the preceding claims, **characterized in that** it is free from carbomers.

7. Use according to at least one of the preceding claims, **characterized in that** they comprise 1,3-dihydroxyacetone as self-tanning substance.

8. Use according to at least one of the preceding claims, **characterized in that** they comprise organic and/or inorganic sunscreen filters.

9. Use according to at least one of the preceding claims, **characterized in that** they comprise inorganic and/or organic and/or modified inorganic pigments.

10. Use of a stabilized cosmetic preparation according to at least one of the preceding claims for colouring the skin of multicellular organisms, in particular the skin of people and animals.

11. Use of a stabilized cosmetic preparation according to Claim 10 for adjusting the colour of differently pigmented areas of skin.

## Revendications

1. Utilisation d'au moins un silicate lamellaire ou d'une association d'au moins un silicate lamellaire et de gomme xanthane pour la stabilisation de préparations cosmétiques contenant de l'eau, qui contiennent une ou plusieurs substances autobronzantes et éventuellement d'autres actifs, adjuvants et additifs cosmétiques et/ou dermatologiques, et qui consistent en des émulsions H/E, des hydrodispersions, des émulsions de Pickering ou des hydrogels.

2. Utilisation selon la revendication 1, **caractérisée en ce que** les préparations contiennent une ou plusieurs substances autobronzantes à une concentration de 0.1 à 10 % en poids et de façon particulièrement préférée de 0,5 à 6 % en poids, chaque fois par rapport au poids total de la préparation.

3. Utilisation selon au moins l'une des revendications précédentes, **caractérisée en ce que** les préparations contiennent un ou plusieurs silicates lamellaires modifiés et/ou minéraux argileux à une concentration totale de 0,05 à 10,0 % en poids, de préférence de 0,1 à 5 % en poids, de façon particulièrement préférée de 1,0 à 3,0 % en poids, chaque fois par rapport au poids total de la préparation.

4. Utilisation selon au moins l'une des revendications précédentes, **caractérisée en ce que** les préparations ont une teneur en émulsifiant inférieure à 1 % en poids, par rapport au poids total des préparations.

5. Utilisation selon au moins l'une des revendications précédentes, **caractérisée en ce que** les préparations sont pratiquement exemptes d'émulsifiant.

6. Utilisation selon au moins l'une des revendications précédentes, **caractérisée en ce que** les préparations sont exemptes de carbomères.

7. Utilisation selon au moins l'une des revendications précédentes, **caractérisée en ce que** les préparations contiennent en tant que substance autobronzante de la 1,3-dihydroxyacétone.

8. Utilisation selon au moins l'une des revendications précédentes, **caractérisée en ce que** les préparations contiennent des filtres antisolaires organiques et/ou inorganiques.

9. Utilisation selon au moins l'une des revendications précédentes, **caractérisée en ce que** les préparations contiennent des pigments organiques et/ou des pigments inorganiques et/ou des pigments inorganiques modifiés.

10. Utilisation d'une préparation cosmétique stabilisée selon au moins l'une des revendications précédentes, pour la coloration de la peau d'organismes pluricellulaires, en particulier de la peau de l'homme et de l'animal.

11. Utilisation d'une préparation cosmétique stabilisée selon la revendication 10, pour l'égalisation de zones de peau différemment pigmentées.
